# EUROPÄISCHE PATENTSCHRIFT

(11) **EP 0 546 397 B1**
(45) Veröffentlichungstag und Bekanntmachung des Hinweises auf die Patenterteilung: **11.10.1995**
(21) Anmeldenummer: 92120260.2
(22) Anmeldetag: 27.11.1992
(51) Int. Cl.: C07D 309/08

(54) **Verfahren zur Reinigung von Tetrahydropyran-4-carbonsäureestern**
Method for the purification of tetrahydropyran-4-carboxylic esters
Méthode pour la purification des esters tétrahydropyrane-4-carboxyliques

(30) Priorität: 13.12.1991 DE 4141221
(43) Veröffentlichungstag der Anmeldung: 16.06.1993
(73) Patentinhaber: BASF Aktiengesellschaft, 67063 Ludwigshafen (DE)
(72) Erfinder: Fischer, Rolf, Dr., W-6900 Heidelberg (DE); Goetz, Norbert, Dr., W-6520 Worms 1 (DE); Kuekenhoehner, Thomas, Dr., W-6737 Boehl-Iggelheim (DE); Rust, Harald, W-6739 Neustadt (DE); Schnurr, Werner, Dr., W-6719 Herxheim (DE)

(56) Entgegenhaltungen:
- EP-A- 0 284 969

## Beschreibung

Diese Erfindung betrifft ein Verfahren zur Reingewinnung von Tetrahydropyran-4-carbonsäureestern, wie sie bei der Umsetzung von 3-(2-Hydroxyethyl)-und 3-(2-Acyloxyethyl)-butyrolactonen mit niederen Alkoholen in Gegenwart von oxidischen Katalysatoren gebildet werden.

Tetrahydropyran-4-carbonsäureester sind wichtige Zwischenprodukte für die Herstellung von 4-Formyl-tetrahydropyran.

Es ist bekannt, daß sich 3-(2-Hydroxyethyl)-butyrolacton mit Methanol zu Tetrahydropyran-4-carbonsäureestern umsetzen läßt (EP-A-284 969). Bei dieser Umsetzung fällt ein Gemisch an, das Tetrahydropyran-4-carbonsäureester, 3-(2-Hydroxyethyl)-butyrolacton, 3-(2-Methoxyethyl)-butyrolacton, 3-Spiro-cyclopropyl-butyrolacton, Methanol, gegebenfalls Wasser, gegebenenfalls Essigsäure und gegebenenfalls Essigsäuremethylester enthält, das bei der Aufarbeitung Probleme bereitet.

Es bestand die Aufgabe, ein Verfahren zur Reingewinnung von Tetrahydropyran-4-carbonsäureester aus derartigen Gemischen zu entwickeln.

Demgemäß wurde ein neues und verbessertes verfahren zur Reinigung von Tetrahydropyran-4-carbonsäurestern der allgemeinen Formel I 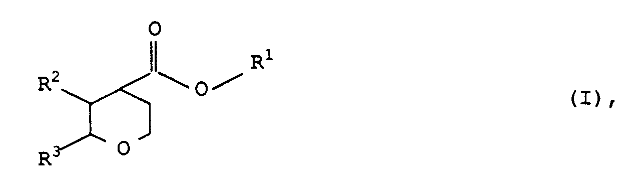
in der
R¹ bis R³ C₁- bis C₄-Alkyl, und R² und R³ zusätzlich Wasserstoff bedeuten,
aus Gemischen, die bei der Umsetzung von Butyrolactonen der allgemeinen Formel II 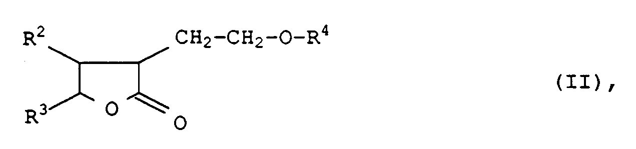
in der
R² und R³ die obengenannte Bedeutung besitzen und R⁴ ein Wasserstoffatom, eine Alkylgruppe mit 1 bis 6 Kohlenstoffatomen oder eine Acylgruppe der Formel -CO-R² bedeuten, mit Alkoholen der Formel R¹OH in Gegenwart von oxidischen Katalysatoren entstehen, durch Destillation, dadurch gekennzeichnet, daß man
a) in einer ersten Kolonne mit einer theoretischen Bodenzahl von 5 bis 25 bei einem Kopfdruck von 700 bis 1100 mbar und einer Kopftemperatur von 50 bis 80°C einen Alkohol und bis zu 10 % des Wassers über Kopf abtrennt,
b) das Sumpfprodukt der ersten Kolonne in eine zweite Kolonne mit einer theoretischen Bodenzahl von 18 bis 40 überführt, welcher zwischen dem 15. und 30. Boden ein Wasserschlepper in Kreisfahrweise zudosiert wird, die bei einem Kopfdruck von 35 bis 350 mbar und einer Kopftemperatur von 18 bis 70°C arbeitet, wobei zwischen dem 8. und 18. Boden bei einer Temperatur von 90 bis 150°C die Tetrahydropyran-4-carbonsäurester ausgeschleust werden, und gegebenenfalls
c) das Sumpfprodukt der zweiten Kolonne in eine dritte Kolonne mit einer theoretischen Bodenzahl von 5 bis 25 einspeist und bei einem Kopfdruck von 1 bis 100 mbar und einer Kopftemperatur von 90 bis 140°C die Kopfprodukte in die Tetrahydropyran-4-carbonsäurester-Synthese zurückführt.

Die für das neue Verfahren eingesetzten Tetrahydropyran-4-carbonsäureester I Rohprodukte enthaltenden Gemische erhält man z.B. bei der Umlagerung von 3-(2-Hydroxyethyl)-butyrolacton II mit Methanol. Sie haben z.B. die folgende Zusammensetzung: 15 bis 30 Gew.-% Tetrahydropyran-4-carbonsäureester, 15 bis 25 Gew.-% Methanol, 5 bis 25 Gew.-% Wasser, 5 bis 20 Gew.-% 3-(2-Methoxyethyl)-butyrolacton, 5 bis 15 Gew.-% 3-Spiro-cyclopropyl-butyrolacton, 0,05 bis 15 Gew.-% 3-(2-Hydroxyethyl)-butyrolacton und 0,1 bis 3 Gew.-% Hochsieder.

Das neue Verfahren wird unter Verwendung von drei Kolonnen z.B. wie folgt durchgeführt:
In einer ersten Kolonne, die eine theoretische Bodenzahl von 5 bis 25 aufweist, wird aus dem Gemisch 95 bis 100 % des Alkohols R¹-OH und bis zu 10 % des im Gemisch enthaltenen Wassers über Kopf abdestilliert. Das Gemisch läßt sich in die Tetrahydropyran-4-carbonsäureester-Synthese zurückführen. Die Temperaturen und Drücke am Kopf betragen 50 bis 80°C und 700 bis 1100 mbar. Das Rücklaufverhältnis beträgt 1 bis 10.

Der Sumpf der ersten Kolonne, der Tetrahydropyran-4-carbonsäureester, 3-(2-Methoxyethyl)-butyrolacton, 3-Spiro-cyclopropyl-butyrolacton, 3-(2-Hydroxyethyl)-butyrolacton, Wasser und gegebenfalls verbliebenes Alkohols R¹-OH sowie Hochsieder enthält, wird in die zweite Kolonne mit einer theoretischen Bodenzahl von 18 bis 40 eingespeist. Zusätzlich wird zwischen Boden 15 und 30 ein Wasserschlepper, gegebenfalls Xylol, zudosiert. Bei einem Kopfdruck von 35 bis 350 mbar und einer Kopftemperatur von 18 bis 70°C erhält man ein Kopfprodukt, das aus Wasser und dem Wasserschlepper besteht. Über einen Phasenscheider wird der Wasserschlepper in die Kolonne und Wasser in die Tetrahydropyran-4-carbonsäureester-Synthese zurückgeführt. Das Rücklaufverhältnis liegt zwischen 1 und 9. Zwischen Boden 8 und 18 wird bei einer Temperatur von 90 bis 150°C Tetrahydropyran-4-carbonsäureester ausgeschleust.

Der Sumpf der zweiten Kolonne, der 3-(2-Methoxyethyl)-butyrolacton, 3-Spiro-cyclopropyl-butyrolacton und 3-(2-Hydroxyethyl)-butyrolacton sowie Hochsieder enthalten kann, wird in eine dritte Kolonne mit einer theoretischen Bodenzahl von 5 bis 25 eingespeist, in der 3-(2-Methoxyethyl)-butyrolacton, 3-Spiro-cyclopropyl-butyrolacton und 3-(2-Hydroxyethyl)-butyrolacton über Kopf abgetrennt und gegebenfalls in die Tetrahydropyran-4-carbonsäureester-Synthese zurückgeführt werden können. Die Temperaturen und Drücke am Kopf der dritten Kolonne liegen bei 90 bis 140°C und 1 und 100 mbar. Das Rücklaufverhältnis liegt z.B. bei 1 bis 10.

Zur Abtrennung des Wassers in der zweiten Kolonne wird ein Wasserschlepper eingesetzt. Als Wasserschlepper können z.B. Cyclohexan, Cyclohexen, Benzol, Toluol, Hexan, Heptan, CCl₄, Chloroform, Acetonitril, Pyridin oder Piperidin, insbesondere Xylole oder das Isomerengemisch der Xylole verwendet werden.

Führt man die Umlagerung zu Tetrahydropyran-4-carbonsäureester mit Estern wie z.B. 3-(2-Acetoxyethyl)-butyrolacton an Stelle von 3-(2-Hydroxyethyl)-butyrolacton durch, so ändert sich die Zusammensetzung des Austrags wie folgt: 15 bis 30 Gew.-% Tetrahydropyran-4-carbonsäureester, 15 bis 30 Gew.-% Methanol, 5 bis 15 Gew.-% Methylacetat, 5 bis 25 Gew.-% Wasser, 2 bis 10 Gew.-% Essigsäure, 5 bis 20 Gew.-% 3-(2-Methoxyethyl)-butyrolacton, 2 bis 15 Gew.-% 3-Spirocyclopropyl-butyrolacton, 0,05 bis 10 Gew.-% 3-(2-Hydroxyethyl)-butyrolacton/3-(2-Acetoxyethyl)-butyrolacton und 0,1 bis 3 Gew.-% Hochsieder.

Die Aufarbeitung verläuft analog zu dem beschriebenen Verfahren mit dem Unterschied, daß bei der ersten Kolonne neben Methanol auch Methylacetat über Kopf abgetrennt wird. Die mengenmäßig geringen Anteile Essigsäure, die im Gemisch enthalten sein können, werden in der zweiten Kolonne mit dem Wasser abgetrennt.

Sollen die Nebenprodukte nicht in die Synthesestufe zurückgeführt werden, so lassen sich 3-(2-Methoxyethyl)-butyrolacton und 3-Spirocyclopropyl-butyrolacton destillativ in einer weiteren Kolonne trennen.

R¹, R² und R³ bedeuten unabhängig voneinander C₁- bis C₄-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl, sowie R² und R³ zusätzlich besonders bevorzugt Wasserstoff.

R⁴ bedeutet C₁- bis C₆-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl, sowie Wasserstoff und -CO-R².

Nach diesem Verfahren lassen sich Tetrahydropyran-4-carbonsäureester besonders vorteilhaft aus den Ausgangsgemischen abtrennen. Durch die azeotrope Abtrennung des Wassers läßt sich ein Destillationsschritt einsparen, da man die Tetrahydropyran-4-carbonsäureester in der gleichen Kolonne seitlich austragen kann. Prinzipiell lassen sich auch höhere Carbonester (C₁-C₄) nach diesem Verfahren gewinnen.

### Beispiel

Es wurde durch katalytische Umlagerung von 3-(2-Acetoxyethyl)-butyrolacton erhaltenes Reaktionsgemisch destilliert, das die folgende Zusammensetzung aufwies:
23,8Gew.-% Tetrahydropyran-4-carbonsäureester,
3,3 Gew.-% 3-Spiro-cyclopropyl-butyrolacton,
7,9 Gew.-% 3-(2-Methoxyethyl)-butyrolacton,
2,4 Gew.-% 3-(2-Hydroxyethyl)-butyrolacton,
5,2 Gew.-% 3-(2-Acetoxyethyl)-butyrolacton,
2,5 Gew.-% Essigsäure,
8,5 Gew.-% Methylacetat,
26,3Gew.-% Methanol und
15,9Gew.-% Wasser.

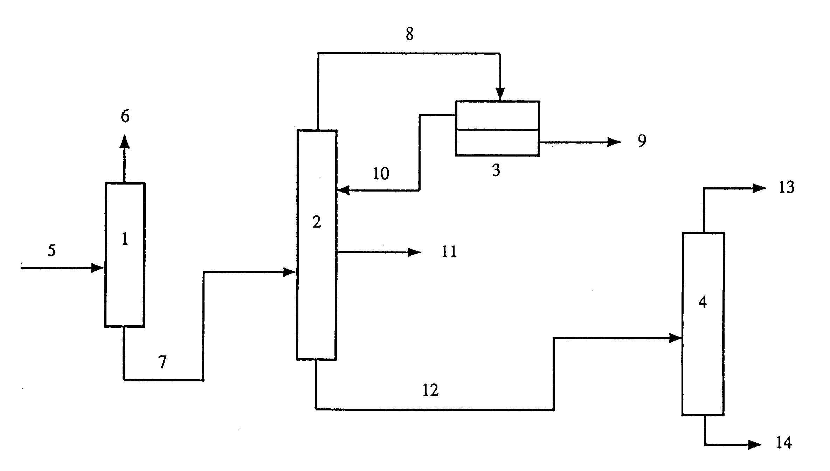

Das Schema der verwendeten Destillationsvorrichtung ist aus der Zeichnung ersichtlich. Über die Zuleitung (5) werden 1000 Teile des Gemisches in die erste Kolonne (1) eingeleitet. Die Kolonne hat 10 theoretische Böden. Bei einer Kopftemperatur von 63°C und einem Kopfdruck von 900 mbar erhält man bei einem Rücklaufverhältnis von 320 Teile eines Kopfproduktes (6) mit der Zusammensetzung 74 Gew.-% Methanol, 20,7 Gew.-% Methylacetat und 3,2 Gew.-% Wasser. Als Sumpfprodukt werden 680 Teile eines Gemisches aus 33,6 Gew.-% Tetrahydropyran-4-carbonsäureester, 4,8 Gew.-% 3-Spiro-cyclopropyl-butyrolacton, 11.9 Gew.-% 3-(2-Methoxyethyl)-butyrolacton, 3,9 Gew.-% 3-(2-Hydroxyethyl)-butyrolacton, 6,3 Gew.-% 3-(2-Acetoxyethyl)-butyrolacton, 3 Gew.-% Essigsäure, 0,1 Gew.-% Methanol und 17.5 Gew.-% Wasser erhalten.

100 Teile Sumpfprodukt (7) aus der ersten Kolonne (1) werden in die zweite Kolonne (2) mit 40 theoretischen Böden geleitet. Als Wasserschlepper werden 0,1 l Xylol (Isomerengemisch) zugesetzt, welches über einen Phasenscheider (3) kontinuierlich in die Kolonne zurückgeführt wird (10). Bei einer Kopftemperatur von 28°C, einem Kopfdruck von 70 mbar und einer Aufkochrate von 1,6 erhält man 23 Teile Kopfprodukt (9) der Zusammensetzung 80,1 Gew.-% Wasser, 11,2 Gew.-% Essigsäure, 3,3 Gew.-% Methanol, 1,8 Gew.-% Tetrahydropyran-4- carbonsäureester und 44 Teile Sumpfprodukt (12) mit der Zusammensetzung 0,1 Gew.-% Tetrahydropyran-4-carbonsäureester, 10,7 Gew.-% 3-Spiro-cyclopropyl-butyrolacton, 28,9 Gew.-% 3-(2-Methoxy- ethyl)-butyrolacton, 6,1 Gew.-% 3-(2-Hydroxyethyl)-butyrolacton und 15,1 Gew.-% 3-(2-Acetoxyethyl)-butyrolacton. Bei einer Temperatur von 108°C werden 33 Teile als Seitenstrom (11) am Boden 17 mit der Zusammensetzung 99,5 Gew.-% Tetrahydropyran-4-carbonsäureester ausgeschleust.

100 Teile Sumpfprodukt (12) aus Kolonne 2 werden in die dritte Kolonne (4) geleitet. Bei einer Kondensattemperatur von 128°C und einem Kopfdruck von 3 mbar erhält man 61 Teile Kopfprodukt (13) und 29 Teile Sumpfprodukt (14) in Form von nicht identifizierten Hochsiedern.

## Patentansprüche

1. Verfahren zur Reinigung von Tetrahydropyran-4-carbonsäureestern der allgemeinen Formel I 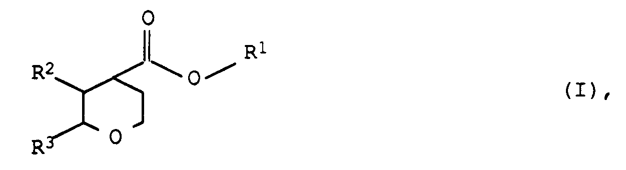 in der
R¹ bis R³ C₁- bis C₄-Alkyl, und
R² und R³ zusätzlich Wasserstoff
bedeuten, aus Gemischen, die bei der Umsetzung von Butyrolactonen der allgemeinen Formel II 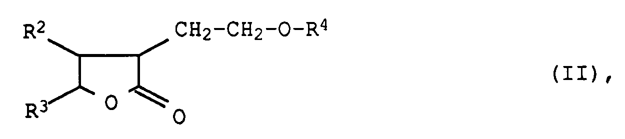 in der
R² und R³ die obengenannte Bedeutung besitzen und R⁴ ein Wasserstoffatom, eine Alkylgruppe mit 1 bis 6 Kohlenstoffatomen oder eine Acylgruppe der Formel -CO-R² bedeuten, mit Alkoholen der Formel R¹OH in Gegenwart von oxidischen Katalysatoren entstehen, durch Destillation, dadurch gekennzeichnet, daß man
a) in einer ersten Kolonne mit einer theoretischen Bodenzahl von 5 bis 25 bei einem Kopfdruck von 700 bis 1100 mbar und einer Kopftemperatur von 50 bis 80°C einen Alkohol und bis zu 10 % des Wassers über Kopf abtrennt,
b) das Sumpfprodukt der ersten Kolonne in eine zweite Kolonne mit einer theoretischen Bodenzahl von 18 bis 40 überführt, welcher zwischen dem 15. und 30. Boden ein Wasserschlepper in Kreisfahrweise zudosiert wird, die bei einem Kopfdruck von 35 bis 350 mbar und einer Kopftemperatur von 18 bis 70°C arbeitet, wobei zwischen dem 8. und 18. Boden bei einer Temperatur von 90 bis 150°C die Tetrahydropyran-4-carbonsäurester ausgeschleust werden, und gegebenenfalls
c) das Sumpfprodukt der zweiten Kolonne in eine dritte Kolonne mit einer theoretischen Bodenzahl von 5 bis 25 einspeist und bei einem Kopfdruck von 1 bis 100 mbar und einer Kopftemperatur von 90 bis 140°C die Kopfprodukte in die Tetrahydropyran-4-carbonsäurester-Synthese zurückführt.

## Claims

1. A process for purifying esters of tetrahydropyran-4-carboxylic acid of the formula I 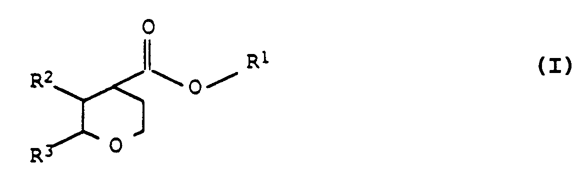 where
R¹ to R³ are each C₁-C₄-alkyl, and R² and R³ are each additionally hydrogen, from mixtures produced in the reaction of butyrolactones of the formula II 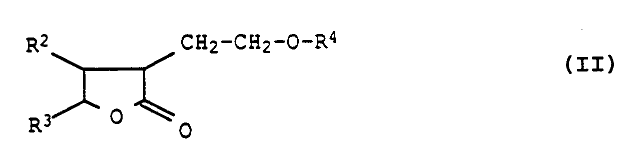 where
R² and R³ have the abovementioned meanings, and R⁴ is hydrogen, alkyl of 1-6 carbons or acyl of the formula -CO-R², with alcohols of the formula R¹OH in the presence of oxide catalysts, by distillation, which comprises
a) removing overhead, in a first column with 5-25 theoretical plates with a distillate pressure of 700-1100 mbar and a distillate temperature of 50-80°C, an alcohol and up to 10% of the water,
b) transferring the bottom product from the first column into a second column with 18-40 theoretical plates, into which a water entrainer is metered between plates 15 and 30, and is circulated, and which operates with a distillate pressure of 35-350 mbar and a distillate temperature of 18-70°C, with the esters of tetrahydropyran-4-carboxylic acid being removed between plates 8 and 18 at 90-150°C, and, where appropriate,
c) feeding the bottom product from the second column into a third column with 5-25 theoretical plates, and returning the overhead products at a distillate pressure of 1-100 mbar and a distillate temperature of 90-140°C to the synthesis of the esters of tetrahydropyran-4-carboxylic acid.

## Revendications

1. Procédé de purification d'esters de l'acide tétrahydropyranne-4-carboxylique de la formule générale I 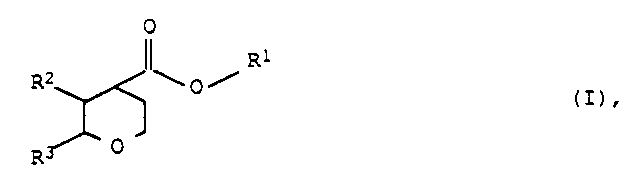 dans laquelle
les symboles R¹ à R³ représentent des radicaux alkyle en C₁ à C₄ et
les symboles R² et R³ représentent, en outre, des atomes d'hydrogène,
à partir de mélanges qui se forment au cours de la réaction de butyrolactones de la formule générale II 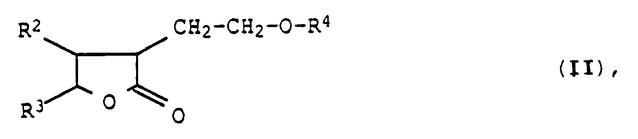 dans laquelle
les substituants R² et R³ possèdent les significations qui leur ont été attribuées ci-dessus et R⁴ représente un atome d'hydrogène, un radical alkyle comportant de 1 à 6 atomes de carbone, ou un radical acyle de la formule -CO-R², avec des alcools de la formule R¹OH, en présence de catalyseurs oxydiques, par distillation, caractérisé en ce que
a) dans une première colonne comportant un nombre de plateaux théorique de 5 à 25, on sépare un alcool et jusqu'à 10% de l'eau à une pression de tête de 700 à 1100 mbars et à une température de tête de 50 à 80°C,
b) on convertit le produit de fond de la première colonne, dans une seconde colonne comportant un nombre de plateaux théorique de 18 à 40 que l'on introduit en quantité dosée à la manière d'un recyclage entre le quinzième et le trentième plateaux d'un appareil d'entraînement d'eau, qui travaille à une pression de tête de 35 à 350 mbars et une température de tête de 18 à 70°C, où entre le huitième et le dix-huitième plateaux, on évacue les esters de l'acide tétrahydropyrnnane-4-carboxylique, à une température de 90 à 150°C et, éventuellement,
c) on injecte le produit de fond de la seconde colonne dans une troisième colonne comportant un nombre de plateaux théorique de 5 à 25 et on renvoie les produits de tête à la synthèse des esters de l'acide tétrahydropyranne-4-carboxylique, à une pression de tête de 1 à 100 mbars et à une température de tête de 90 à 140°C.
